# EUROPEAN PATENT APPLICATION

(11) **EP 2 779 355 A1**
(43) Date of publication of application: **17.09.2014**
(21) Application number: 14155166.3
(22) Date of filing: 14.02.2014
(51) Int. Cl.: H02J 7/00, A61B 17/16

(54) **Charging assemblies for maintaining sterility of surgical instrument batteries during charging**

(30) Priority: 14.03.2013 US 201361784424 P; 07.01.2014 US 201414148860
(71) Applicant: Covidien LP, Mansfield, MA 02048 (US)
(72) Inventor: Lopez, John T., Boulder, CO Colorado 80301 (US); Schaning, Matthew, Cambridge, WI Wisconsin 53523 (US)
(74) Representative: HOFFMANN EITLE

(57) **Abstract**

A charging assembly includes a container and an external charging portion. The container includes a base and a cover. The base defines an interior volume. The cover is releasably engagable with the base for enclosing the interior volume. The container is configured to maintain the interior volume in a sterile condition. At least one electrical contact is disposed within the interior volume of the base. The external charging portion is operably couplable to the container for charging a battery assembly positioned within the interior volume of the base via the at least one electrical contact.

## Description

### BACKGROUND

### Technical Field

The present disclosure relates to battery charging and, more particularly, to charging assemblies for maintaining sterility of surgical instrument batteries during charging of such batteries.

### Background of Related Art

Battery-powered surgical instruments are advantageous in that they obviate the need for cables coupling the device to an electrical outlet or external power source. A typical rechargeable battery pack for a battery-powered surgical instrument includes a housing containing one or more battery cells coupled to one another via a powering circuit through which the battery pack is able to provide electrical power to the surgical instrument and receive electrical power from a charger.

Maintaining sterility in a surgical environment reduces the likelihood of infection and helps prevent the spread of disease. In order to maintain a sterile surgical environment, surgical instrumentation is sterilized and maintained in sterile condition prior to entering the sterile surgical environment. Reusable surgical instruments, or reusable components of surgical instruments, are thus required to be sterilized, e.g., via autoclaving or using a Sterrad® system, after each use and/or prior to re-entering the sterile surgical environment. Rechargeable battery packs additionally require charging after each use or several uses.

### SUMMARY

As used herein, the term "distal" refers to the portion that is being described which is further from a user, while the term "proximal" refers to the portion that is being described which is closer to a user. Further, to the extent consistent, any of the aspects described herein may be used in conjunction with any or all of the other aspects described herein.

In accordance with aspects of the present disclosure, a charging assembly is provided. The charging assembly includes a container and an external charging portion. The container includes a base defining an interior volume and a cover. The cover is releasably engagable with the base for enclosing the interior volume. The container is configured to maintain the interior volume in a sterile condition. At least one electrical contact is disposed within the interior volume of the base. The external charging portion is operably couplable to the container for charging a battery assembly positioned within the interior volume of the base via the at least one electrical contact.

In aspects, the container includes at least one charging bay disposed within the interior volume. The at least one charging bay is configured to receive a battery assembly.

In aspects, the base of the container is configured for wireless power transmission with the external charging portion. More specifically, the base of the container may include a support member incorporating an inductive coil and the external charging portion may include an inductive charging mat. The support member and charging mat are configured for wireless power transmission therebetween.

In aspects, the container includes an exterior receptacle for coupling the container to the external charging portion. More specifically, the charging assembly may further include a connector cable configured to couple between the exterior receptacle of the container and the external charging portion for transmitting power therebetween.

In aspects, the container is formed from a sterilizable material. In particular, the container may be configured for hydrogen peroxide sterilization.

A system provided in accordance with the present disclosure includes a surgical instrument, a battery assembly, and a charging assembly. The battery assembly is releasably engagable with the surgical instrument for powering the surgical instrument. The battery assembly is sterilizable. The charging assembly includes a container having a base configured to receive the battery assembly and a cover releasably engagable with the base for enclosing the battery assembly within the container. The container is configured to maintain the battery assembly in a sterile condition. The charging assembly further includes an external charging portion. The external charging portion is operably couplable to the container for charging the battery assembly.

In aspects, the battery assembly includes at least one electrical contact through which the battery assembly is charged and discharged.

In aspects, the container further includes at least one electrical contact configured to couple to the at least one electrical contact of the battery assembly for charging the battery assembly.

In aspects, the container includes a charging bay configured to receive the battery assembly.

In aspects, the base of the container is configured for wireless power transmission with the external charging portion. Alternatively, the container may include an exterior receptacle for coupling the container to the external charging portion.

In aspects, the container and battery assembly are sterilizable with the battery assembly disposed within the container. In particular, the container and battery assembly may be sterilizable via hydrogen peroxide sterilization.

A method provided in accordance with aspects of the present disclosure includes performing at least one surgical task with a battery-powered surgical device, disengaging a battery assembly from the battery-powered surgical device, sterilizing the battery assembly, sterilizing a container, enclosing the battery assembly within the container, electrically coupling an external charging portion to the container for charging the battery assembly, and returning the sterile battery assembly to a sterile environment.

In aspects, the step of enclosing the battery assembly within the container is performed before sterilizing the battery assembly and sterilizing the container such that the battery assembly and container are sterilized with the battery assembly enclosed within the container.

In aspects, the step of coupling the external charging portion to the container for charging the battery assembly includes approximating wireless power transmission components of the external charging portion and container relative to one another.

In aspects, the step of coupling the external charging portion to the container for charging the battery assembly includes coupling a connector cable between the container and the external charging portion.

In an embodiment of the method, the battery assembly is sterilized prior to charging the battery assembly.

In an embodiment of the various aspects, the container is configured for maintaining the battery assembly in a sterile condition during a charging process.

In an embodiment of the method aspects, the container maintains the battery assembly in a sterile condition during a charging process.

In embodiments of the various aspects, the external charging portion includes a power cord having a plug at the free end thereof for coupling the external charging portion to a standard outlet.

In embodiments of the method aspects, the battery assembly is positioned within the container and the cover of the container is engaged about the base of the container in a sterile environment so as not to expose the battery assembly or the interior of the container to unsterile conditions and, thereafter, the container is removed from the sterile environment without compromising the sterility of the battery assembly as the battery assembly is enclosed within the container.

In an embodiment, the method comprises, with the battery assembly enclosed within the container, coupling the container to the external charging portion to charge the battery assembly, wherein the external charging portion is non-sterile yet the battery assembly is charged without compromising the sterility of the battery assembly because it is enclosed within the container which maintains the battery assembly in a sterile environment/condition.

In embodiments of the apparatus or system aspects, the battery assembly is positionable within the container and the cover is engageable about the base in a sterile environment so as not to expose the battery assembly or the interior of the container to unsterile conditions and the container is configured so as to be removable from the sterile environment without compromising the sterility of the battery assembly because the battery assembly is enclosed within the container and the container is coupleable to the external charging portion to charge the battery assembly, wherein the external charging portion is located outside of the sterile environment.

In the various aspects, the charging assembly includes a plurality of LED's or other indicators associated therewith configured to indicate a charging status and/or charging condition of the battery assembly being charged therein, and wherein the container includes a transparent material to allow visualization of the indicators for monitoring the charging status or condition of the battery assembly.

### BRIEF DESCRIPTION OF THE DRAWINGS

Various aspects of the present disclosure are described hereinbelow with reference to the drawings, wherein:
FIG. 1 is a side, perspective view of a portable, battery-powered surgical instrument configured for use in accordance with the present disclosure;
FIG. 2 is a side, perspective view of another portable, battery-powered surgical instrument configured for use in accordance with the present disclosure;
FIG. 3 is a side, perspective view of a battery assembly provided in accordance with the present disclosure and configured for use with either or both of the instruments of FIGS. 1 and 2;
FIG. 4 is an exploded, perspective view of the battery assembly of FIG. 3;
FIG. 5A is an exploded, perspective view of a sterilization and charging container provided in accordance with the present disclosure;
FIG. 5B is an exploded, perspective view of the sterilization and charging container of FIG. 5A including the battery assembly of FIG. 3 disposed therein;
FIG. 6A is a perspective view of the sterilization and charging container of FIG. 5A shown in conjunction with associated external charging components provided in accordance with the present disclosure;
FIG. 6B is a perspective view of another sterilization and charging container provided in accordance with the present disclosure and shown in conjunction with associated external charging components provided in accordance with the present disclosure;
Fig. 7A is a perspective view of a sterile wrap housing a charging device provided in accordance with the present disclosure; and
FIG. 7B is a side view of the sterile wrap and charging device shown in FIG. 7A.

### DETAILED DESCRIPTION

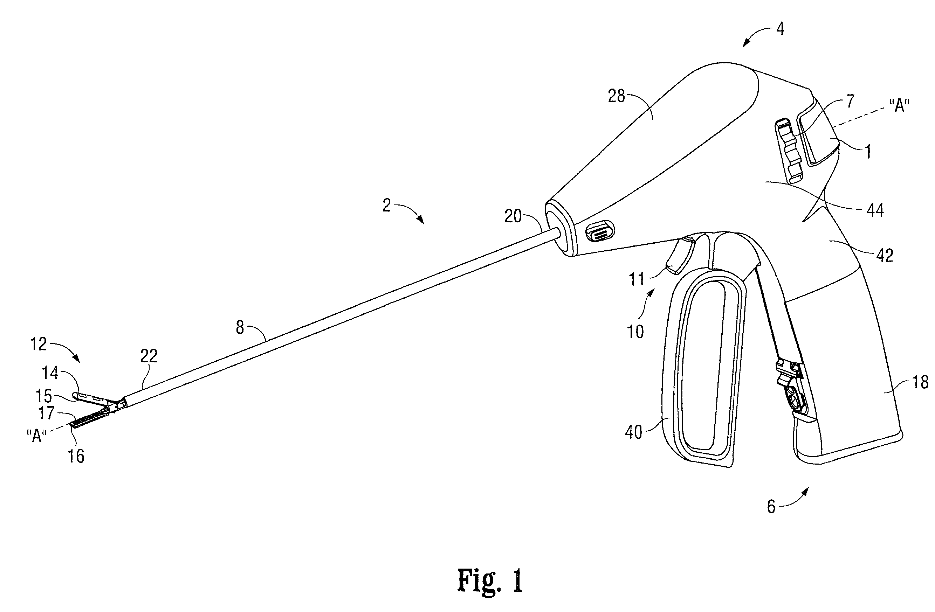

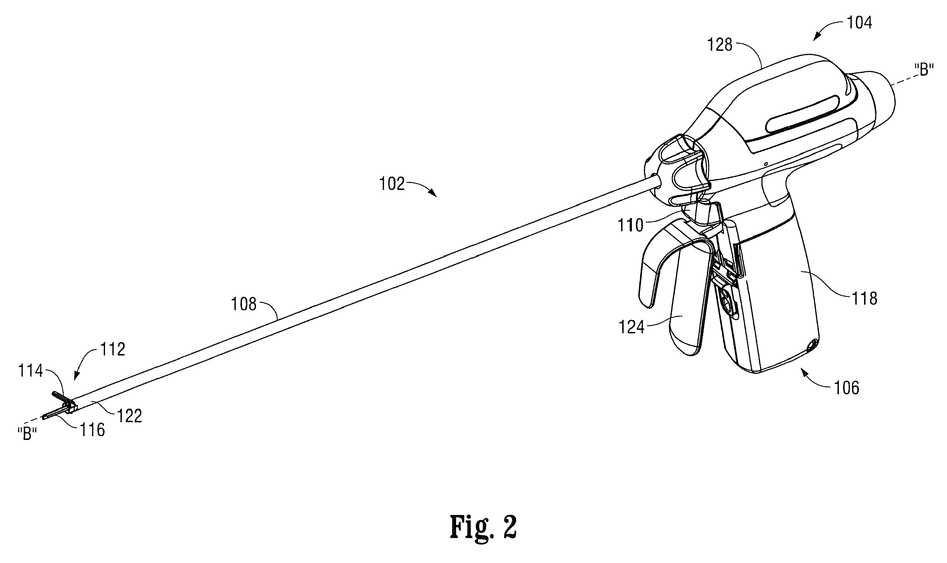

Referring now to FIGS. 1 and 2, FIG. 1 depicts a portable, battery-powered electrosurgical instrument 2 and FIG. 2 depicts a portable, battery-powered ultrasonic surgical instrument 102. For the purposes herein, either an electrosurgical instrument, e.g., instrument 2, an ultrasonic instrument, e.g., instrument 102, or any other suitable battery-powered device, e.g., a surgical instrument, handheld tool, electronic device, or the like, may be utilized in accordance with the present disclosure. Obviously, different considerations apply to each particular type of device; however, the features and aspects of the present disclosure are equally applicable and remain generally consistent with respect to any suitable battery-powered device. For the purposes herein, electrosurgical instrument 2 and ultrasonic instrument 102 are generally described.

With reference to FIG. 1, electrosurgical instrument 2, shown as an electrosurgical forceps, generally includes a housing 4, a battery assembly 18, an electrosurgical generator 28, a handle assembly 6, a rotating assembly 7, a shaft 8, a trigger assembly 10, a drive assembly (not shown), and an end effector assembly 12. End effector assembly 12 operatively connects to handle assembly 6 via the drive assembly (not shown) for imparting movement of one or both of jaw members 14, 16 of end effector assembly 12 between a spaced-apart position and an approximated position for grasping tissue therebetween.

Continuing with reference to FIG. 1, shaft 8 is coupled to housing 4 at proximal end 20 thereof and extends distally from housing 4 to define a longitudinal axis "A-A." End effector assembly 12, including jaw members 14 and 16, is disposed at a distal end 22 of shaft 8. End effector assembly 12 is shown configured as a unilateral assembly wherein jaw member 16 is fixed relative to shaft 8 and jaw member 14 is pivotable relative to jaw member 16 and shaft 8 between the spaced-apart and approximated positions. However, this configuration may be reversed, e.g., wherein jaw member 14 is fixed relative to shaft 8 and jaw member 16 is pivotable relative to jaw member 14 and shaft 8. Alternatively, end effector assembly 12 may be configured as a bilateral assembly, e.g., wherein both jaw members 14, 16 are pivotable relative to one another and shaft 8 between the spaced-apart and approximated positions.

Electrosurgical instrument 2 may be configured as a bipolar instrument. That is, each of the jaw members 14, 16 may include a respective seal plate 15, 17 that is configured to function as an active (or activatable) and/or return electrode. Each seal plate 15, 17 is electrically coupled to generator 28 via one or more electrical leads (not shown) that extend from generator 28, through shaft 8, and eventually coupling to one or both of seal plates 15, 17 for conducting energy through tissue grasped therebetween. However, forceps 2 may alternatively be configured as a monopolar instrument.

Handle assembly 6 includes a moveable handle 40 that is movable relative to fixed handle portion 42 for moving jaw members 14, 16 of end effector assembly 12 between the spaced-apart and approximated positions. Rotating assembly 7 is rotatable in either direction about longitudinal axis "A-A" to rotate shaft 8 and, thus, end effector assembly 12 about longitudinal axis "A-A." Trigger assembly 10 is in operable communication with a knife assembly (not shown) including a knife blade (not shown) that is selectively translatable between jaw members 14, 16 to cut tissue grasped therebetween, e.g., upon actuation of trigger 11 of trigger assembly 10.

With continued reference to FIG. 1, housing 4 is configured to releasably engage electrosurgical generator 28 and battery assembly 18. Generator 28 is releasably engagable with body portion 44 of housing 4, while battery assembly 18 is releasably engagable with fixed handle portion 42 of housing 4. More specifically, battery assembly 18 is configured to engage fixed handle portion 42 of housing 4 such that battery assembly 18 functions as the stationary handle of housing 4 to facilitate grasping of the forceps 2. Generator 28 releasably engages body portion 44 of housing 4 and may be selectively removable from body portion 44 either in connection with the removal of battery assembly 18 or independently.

When forceps 2 is assembled, generator 28 is disposed in operable communication with battery assembly 18 to provide electrosurgical energy to end effector 12 for electrosurgically treating tissue, e.g., to seal tissue, although forceps 2 may alternatively be configured to deliver any other suitable form of energy to tissue, e.g., thermal energy, microwave energy, light energy, etc. With respect to electrosurgical tissue treatment, generator 28 may include suitable electronics that convert the electrical energy from battery assembly 18 into an RF energy waveform to energize one or both of jaw members 14, 16. That is, generator 28 may be configured to transmit RF energy to seal plate 15 of jaw member 14 and/or seal plate 17 of jaw member 16 to conduct energy therebetween for treating tissue. Activation switch 1 disposed on housing 4 is activatable for selectively enabling generator 28 to generate and subsequently transmit RF energy to seal plate 15 and/or seal plate 17 of jaw members 14, 16, respectively, for treating tissue grasped therebetween.

Referring now to FIG. 2, ultrasonic instrument 102 includes components similar to that of forceps 2 shown in Fig. 1, namely, a housing 104, a battery assembly 118, a generator 128, a handle assembly 106, a shaft 108, and an end effector assembly 112. Accordingly, only the difference between ultrasonic instrument 102 and forceps 2 (FIG. 1) will be described in detail below.

Housing 104 is configured to releasably engage ultrasonic generator 128 and battery assembly 118. Shaft 108 extends distally from housing 104 to define longitudinal axis "B-B" and includes end effector assembly 112 disposed at distal end 122 thereof. One or both of jaw members 114 and 116 of end effector assembly 112 are movable relative to one another, e.g., upon actuation of moveable handle 124, between an open position and a clamping position for grasping tissue therebetween. Further, one of the jaw members, e.g., jaw member 116, serves as an active or oscillating ultrasonic blade that is selectively activatable to ultrasonically treat tissue grasped between jaw members 114, 116.

Generator 128 includes a transducer (not shown) configured to convert electrical energy provided by battery assembly 118 into mechanical energy that produces motion at the end of a waveguide, e.g., at blade 116. More specifically, the electronics (not explicitly shown) of the generator 128 convert the electrical energy provided by battery assembly 118 into a high voltage AC waveform that drives the transducer (not shown). When the transducer (not shown) and the waveguide are driven at their resonant frequency, mechanical, e.g., ultrasonic, motion is produced at the active jaw member 116 for treating tissue grasped between jaw members 114, 116. Further, an activation button 110 disposed on housing 104 is selectively activatable to operate instrument 102 in two modes of operation: a low-power mode of operation and a high-power mode of operation.

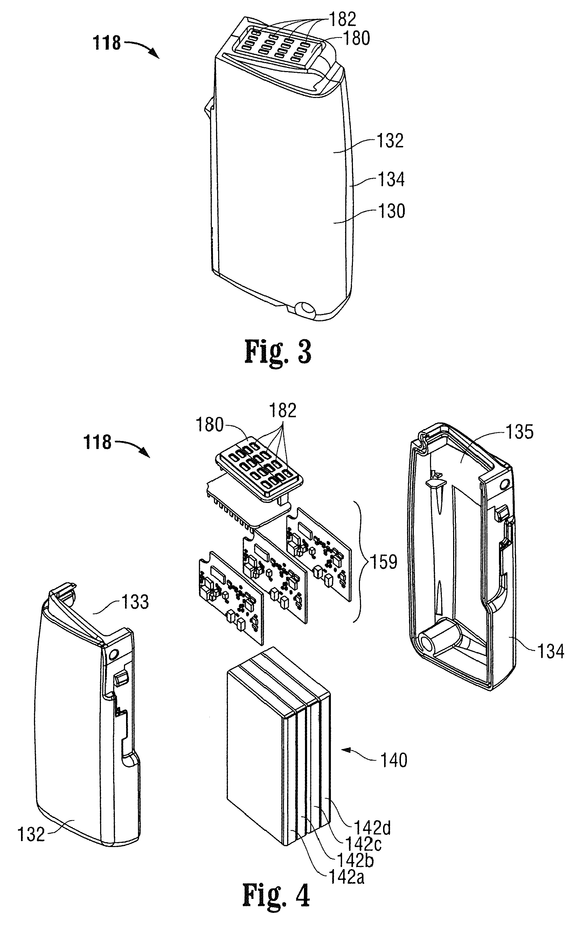

With reference to FIGS. 3-4, battery assembly 118 generally includes an outer housing 130, a battery pack 140, battery circuitry 159, and a contact cap 180. Battery assembly 18 of electrosurgical instrument 2 (FIG. 1) may be configured similarly to battery pack 118 and, thus, will not be described herein for purposes of brevity.

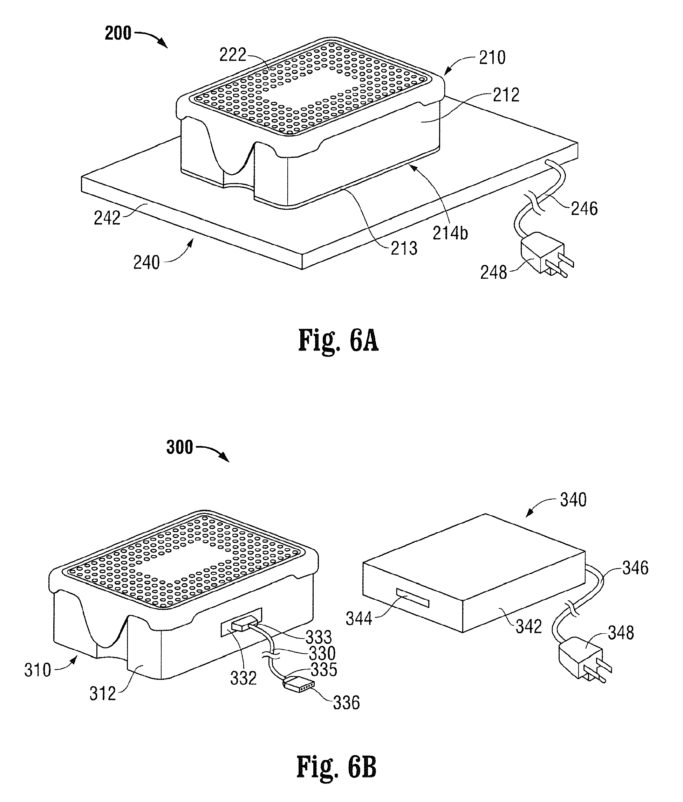

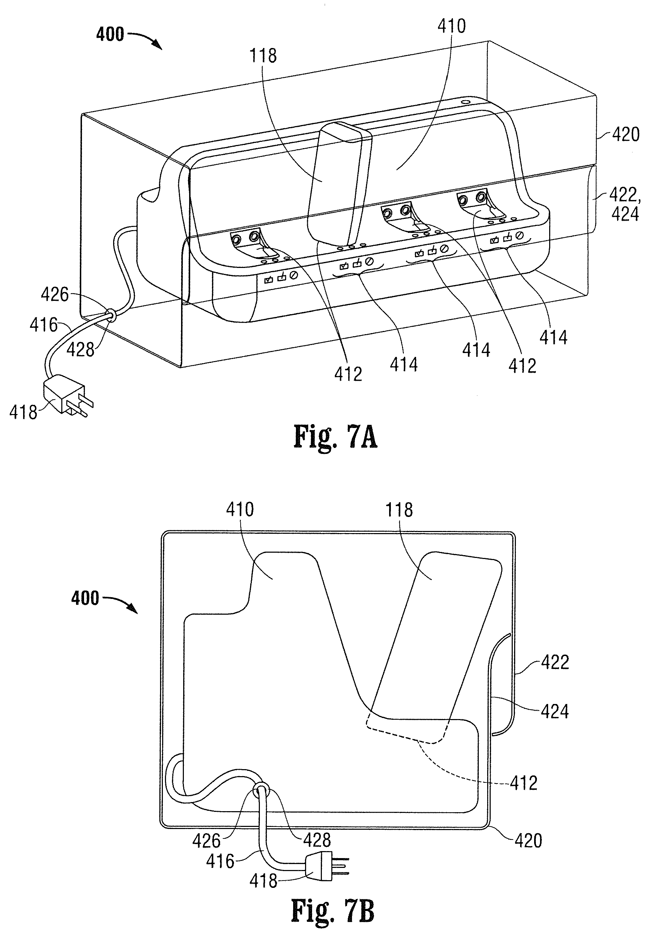

Outer housing 130 of battery assembly 118 is formed from first and second housing parts 132, 134 that cooperate to house battery pack 140 and battery circuitry 159. Housing parts 132, 134 define cut-outs 133, 135, respectively, that cooperate to form a window configured to retain contact cap 180. Contact cap 180 is electrically coupled to battery circuitry 159, which, in turn, is electrically coupled to battery pack 140. Contact cap 180 includes a plurality of contacts 182 configured to provide an electrical interface between battery assembly 118, e.g., battery pack 140 and battery circuitry 159, and both the battery-powered device, e.g., electrosurgical instrument 2 (FIG. 1) or ultrasonic instrument 102 (Fig. 2), and battery charging device, e.g., charging assembly 200 (FIG. 6A), charging assembly 300 (FIG. 6B), or charging assembly 400 (FIGS. 7A-7B), for transmitting power and/or control signals therebetween. Battery pack 140 includes a plurality of battery cell assemblies 142a, 142b, 142c, 142d, e.g., four (4) battery cell assemblies 142a-142d, although greater or fewer battery cell assemblies 142a-142d are also contemplated.

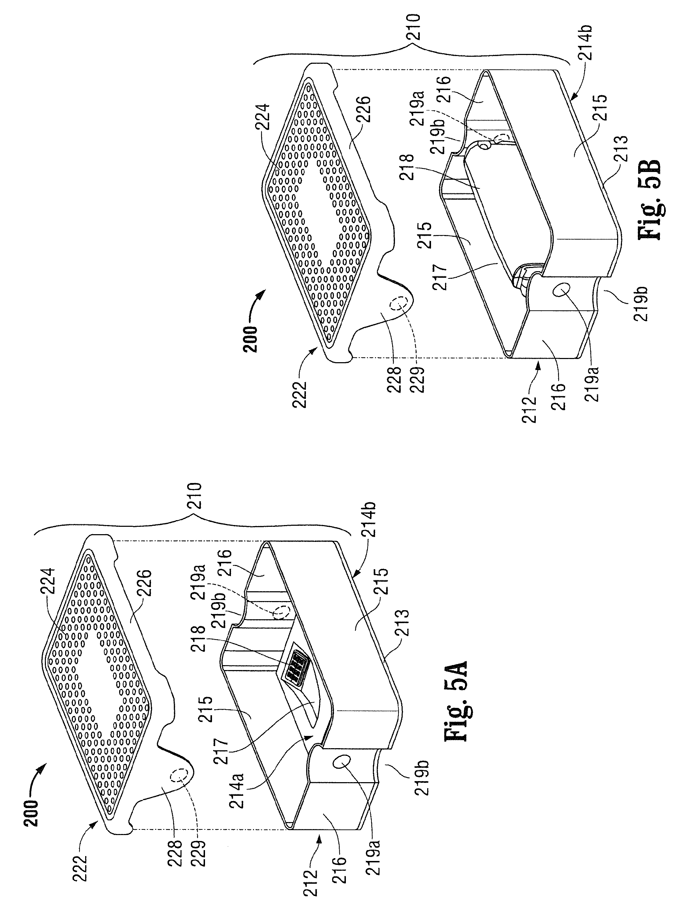

Turning now to FIGS. 5A-7B, with respect to the sterilization of battery assemblies, it has been found that the risk of thermal runaway and/or other damage to the battery assembly is increased in instances where the state of charge of the battery assembly approaches 100%, e.g., when the battery is fully or near-fully charged. Thus, after use, it is desirable to sterilize the battery assembly prior to charging the battery assembly, rather than the other way around. However, sterilizing the battery assembly prior to charging the battery assembly requires that the battery assembly be maintained in sterile condition during the charging process. Accordingly, the present disclosure provides various embodiments of charging assemblies 200, 300, 400 (FIGS. 5A-6A, 6B, and 7A-7B, respectively) configured for charging battery assembly 118 (FIGS. 3-4), or any other suitable rechargeable battery assembly, while maintaining battery assembly 118 (FiGS. 3-4) in a sterile condition and/or allowing for sterilization of battery assembly 118 (FIGS. 3-4). Each of charging assemblies 200, 300, 400 (FIGS. 5A-6A, 6B, and 7A-7B, respectively) will be described in greater detail, in turn, below.

Referring to FIGS. 5A-6A, charging assembly 200 is shown generally including a sterilization and charging container 210 (FIGS. 5A-5B) and an external charging portion 240 (FIG. 6A). Sterilization and charging container 210 generally includes a base 212 and a cover 222 that cooperate to fully enclose and retain battery assembly 118 therein. Container 210 is formed from a sterilizable material, e.g., thermoplastics such as polycarbonate and low-density polyethylene (LDPE), and/or is wrapped in a sterilizable wrapping, e.g., synthetic wrappings such as Tyvek®.

With reference to FIGS. 5A-5B in particular, base 212 of container 210 defines a generally rectangular configuration, although other configurations are contemplated. Base 212 includes a planar support member 213, a pair of opposed long walls 215 and a pair of opposed short walls 216. Long and short walls 215, 216, respectively, are disposed about the outer periphery of planar support member 213 so as to define an interior volume within base 212. Planar support member 213 defines an interior surface 214a, and an exterior surface 214b and incorporates inductive charging components, e.g., an inductive coil, for inductively charging battery assembly 118 upon abutment of exterior surface 214b of planar support member 213 and charging surface 244 of external charging portion 240 (FIG. 6A), as will be described in greater detail below. The inductive charging components may include any suitable components and/or features known in the art. As an alternative to inductive charging, planar support member 213 may include any other suitable wireless charging components know in the art.

A charging bay 217 is disposed on interior surface 214a of planar support member 213 and is configured to at least partially receive battery assembly 118. Charging bay 217, or a portion thereof, may be shaped complementary to battery assembly 118 to retain battery assembly 118 in position within base 212 during transport of container 210. A plurality of contacts 218 electrically coupled to the inductive components of planar support member 213 and configured to electrically mate with two or more of contacts 182 (FIGS. 3-4) of battery assembly 118 are disposed within charging bay 217 such that, upon seating of battery assembly 118 within charging bay 217, contacts 182 (FIGS. 3-4) and contacts 218 electrically mate with one another. It is also envisioned that base 212 of container 210 include a plurality of charging bays 217 for retaining a plurality of battery assemblies 118 therein.

Short walls 216 of base 212 each include one or more engagement features, e.g., protrusions 219a, respectively, configured to releasably engage corresponding engagement features, e.g., dimples 229 of flanges 228 of cover 222. Alternatively or additionally, the engagement features may be disposed on long walls 215. Short walls 216 further define cut-outs 219b configured to facilitate the grasping of base 212 during transport and/or to facilitate engaging and removing cover 222 from base 212.

Continuing with reference to FIGS. 5A-5B, cover 222 of container 210 includes a planar member 224 and an overhang 226 extending about the outer periphery of planar member 224. Planar member 224 is dimensioned similarly to planar support member 213 of base 212 such that, upon positioning of cover 222 about base 212, overhang 226 is disposed about and positioned in abutting relation with walls 215, 216 of base 212 to engage cover 222 about base 212. Overhang 226 of cover 212 includes a pair of flanges 228 extending from opposed sides thereof (only one flange 228 is shown). Flanges 228 are configured to extend along short walls 216 of base 212 and, as mentioned above, each flange 228 includes a dimple 229 configured to receive a respective protrusion 219a of base 212 to releasably engage cover 222 about bases 212. Flanges 228 are resiliently movable relative to cover 222 to permit a user to disengage protrusions 219a from dimples 229 to release cover 222 from base 212. Other suitable releasable engagement mechanisms for engaging cover 222 about base 212 and/or for facilitating the releasable engagement of cover 222 about base 212 are also contemplated.

Turning now to FIG. 6A, in conjunction with FIGS. 5A-5B, external charging portion 240 of charging assembly 200 includes a charging mat 242 defining a charging surface 244. A power cord 246 is coupled to and extends from charging mat 242. Charging mat 242 incorporates indicative charging components, e.g., an inductive coil, or other suitable components for wirelessly providing power to container 210, for charging battery assembly 118. Power cord 246 includes a receptacle 248 for coupling external charging portion 240 to a standard outlet (not shown), although other configurations are also contemplated. External charging portion 240 need not be sterilizable or maintained in sterile condition since, as can be appreciated, battery assembly 118 can be maintained within container 210, in sterile condition, while being charged by external charging portion 240 via the wireless power transfer between charging mat 242 and planar support member 213 of container 210 and, ultimately, the electrically coupled contacts 218, 182 of container 210 and battery assembly 118, respectively.

Turning now to FIG. 6B, another embodiment of a charging assembly provided in accordance with the present disclosure is shown generally identified by reference numeral 300. Charging assembly 300 includes a sterilization and charging container 310 that is similar to and may include any of the features of a sterilization and charging container 210 of charging assembly 200 (FIG. 5A-6A). Accordingly, only the differences between charging assembly 300 and charging assembly 200 (FIGS. 5A-6A) will be described in detail below for purposes of brevity. Charging assembly 300 further includes an external charging portion 340.

Container 310 of charging assembly 300 includes a connector cable 330 extending from one of the walls that forms base 312 of container 310. More specifically, base 312 of container 310 includes a receptacle 332 disposed on an external surface thereof that is configured to engage first end 333 of connector cable 330. First end 333 of connector cable 330 may be releasably engagable with receptacle 332 or may be fixedly engaged thereto. Receptacle 332 is electrically coupled to the contacts disposed within the charging bay (not explicitly shown; similar to contacts 218 and charging bay 217 of container 210 (FIGS. 5A-5B)) of container 310. Further, it is envisioned that connector cable 330, similarly as container 310, be sterilizable, at least in embodiments where connector cable 330 is fixedly engaged to container 310.

Connector cable 330 extends to a second end 335 thereof that includes a plug 336. Plug 336 is configured to releasably engage a slot 344 defined within base member 342 of external charging portion 340 charging assembly 300, although other suitable releasable engagement mechanisms are also contemplated. For example, rather than being fixedly engaged to container, connector cable 330 may be fixedly engaged to external charging portion 340. Base member 342 houses suitable circuitry for transmitting controlling the power supplied to connector cable 330 and, ultimately, to container 310 for charging battery assembly 118 (FIGS. 3-4). Base member 342 further includes a power cord 346 having a plug 348 at the free end thereof for coupling charging portion 340 to a standard outlet (not shown), although other configurations are also contemplated. Similarly as described above with respect to external charging portion 240 (FIG. 6A), external charging portion 340 need not be sterilizable nor maintained in sterile condition.

Referring again to FIGS. 5A-6A, in conjunction with FIGS. 2 and 3, the use and operation of charging assembly 200 for charging battery assembly 118 is described. The use and operation of charging assembly 300 (FIG. 6B) is similar to that of charging assembly 200 and, thus, will not be described herein to avoid unnecessary repetition.

Initially, with battery assembly 118 engaged to ultrasonic instrument 102, ultrasonic instrument 102 is be used to perform one or more surgical tasks during a surgical procedure. At the completion of the surgical procedure, battery assembly 118 is removed from ultrasonic instrument 102 and is sterilized, e.g., via steam sterilization (by placing battery assembly 118 in an autoclave), hydrogen peroxide sterilization (for example, using the Sterrad® system), or other suitable sterilization technique. As mentioned above, sterilizing battery assembly 118 prior to charging reduces the likelihood of thermal runaway or other damage to battery assembly 118. If not already sterilized, container 210 may also be sterilized separately or with battery assembly 118 disposed therein. In one particular embodiment, battery assembly 118 is properly seated within base 212 of container 210 and cover 222 of container 210 may be engaged about base 212 to enclose battery assembly 118 within container 210 prior to sterilization. Thereafter, battery assembly 118 and container 210 may be sterilized together as a unit, without requiring removal of battery assembly 118. In some situations, it may be desirably to clean battery assembly 118, e.g., wipe down battery assembly 118, prior to placing battery assembly 118 within container 210 for sterilization.

Once battery assembly 118 is sterilized, battery assembly 118 is placed in base 212 of container 210 (if not sterilized within container 210), such that battery assembly 118 is properly seated within charging bay 217 with contacts 218 of container 210 and contacts 182 of battery assembly 118 electrically coupled to one another. Thereafter, cover 222 is engaged about base 212 to enclose battery assembly 118 within container 210. The positioning of battery assembly 118 within container 210 and the engagement of cover 222 of container about base 212 of container 210 is performed in a sterile environment so as not to expose battery assembly 118 or the interior of container 210 to unsterile conditions. Thereafter, container 210 may be removed from the sterile environment without compromising the sterility of battery assembly 118, as battery assembly 118 is enclosed within container 210.

With battery assembly 118 enclosed within container 210, container 210 may be placed on charging mat 242 to charge battery assembly 118. As can be appreciated, the non-sterile external charging portion 240 of charging assembly 200 allows for the charging of battery assembly 118 without compromising the sterility of battery assembly 118. Referring additionally to FIG. 6B, with respect to charging assembly 300, cable connector 330 is coupled between base member 342 of external charging portion 340 and container 310 to likewise charge battery assembly 118 without compromising its sterility. Once battery assembly 118 is sufficiently charged, battery assembly 118 can be removed from container 210 in a sterile manner and to the sterile surgical environment, eventually for engagement with a surgical instrument, e.g., a new or sterilized ultrasonic instrument 102 (FIG. 2), for subsequent use of battery assembly 118. Although the use of charging assembly 200 is described above in a particular order, it is envisioned that the above-described steps of use be performed in any suitable order, depending on the circumstances.

Turning now to FIGS. 7A-7B, also provided in accordance with the present disclosure is a charging assembly 400 that generally includes a charging base 410 and a sterile wrap or enclosure 420 sealing the charging base 410 within enclosure 420. Charging base 410 is shown including a plurality of charging bays 412, each of which is configured to receive a battery assembly 118 for charging the battery assembly 118. Each charging bay 412 further includes a plurality of LED's 414 associated therewith and configured to indicate a charging status and/or charging condition of the battery assembly 118 being charged therein. A power cord 416 extends from charging base 410. Power cord 416 includes a plug 418 disposed at the free end thereof for coupling charging base 410 to a standard wall outlet (not shown), although other configurations are also contemplated. Further, charging base 410 may include any additional or alternative features and/or components known in the art to facilitate the charging of battery assemblies, e.g., one or more of battery assemblies 118.

Continuing with reference to FIGS. 7A-7B, enclosure 420 surrounds and encloses charging base 410. Enclosure 420 serves as a sterile barrier to maintain charging base 410 in sterile condition. It is contemplated that charging base 410 be sterilized, e.g., as a last phase of manufacturing, and placed in enclosure 420 during manufacture or, alternatively, that a sterile charging base 410 be positionable within enclosure 420 by the end-user to maintain its sterility.

It is envisioned that enclosure 420 be formed from a transparent material to allow visualization of a charging base 410 for monitoring charging of battery assemblies 118 or other similarly purposes. As best shown in FIG. 7B, enclosure 420 includes first and second flaps 422, 424 that overlap one another in a closed position to fully enclose charging base 410. Flaps 422, 424 are movable from this closed position to an open, spaced-apart position to permit insertion and/or removal of battery assemblies into enclosure 420. Flaps 422, 424 may be retained in the overlapping, closed position via friction or any other suitable mechanism, e.g., magnets, snaps, tongue and groove engagement, etc.

With continued reference to FIGS. 7A-7B, enclosure 420 further includes an aperture 426 defined therethrough that is configured to receive power cord 416 of charging base 410. Aperture 426 allows power cord 416 to extend through enclosure 420 such that charging base 410 may be maintained within enclosure 420 in sterile condition while allowing plug 418 of power cord 416 to extend outside the sterile barrier for engagement with a wall outlet (not shown) for powering charging base 410. Aperture 426 may be surrounded by an O-ring 428 or other suitable sealing member, e.g., a sealing gasket, compression ring, etc., for sealing about power cord 416.

In use, rather than charging battery assembly 118 prior to sterilization which, as mentioned above, may damage battery assembly 118, battery assembly 118 may be sterilized and then transferred in a sterile manner into enclosure 420 and, more specifically, into engagement with one of charging bays 412 of charging base 410. Thus, battery assembly 118 can be charged without compromising the sterility of battery assembly 118.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.

## Claims

1. A charging assembly, comprising:
a container including a base defining an interior volume and a cover, the cover releasably engagable with the base for enclosing the interior volume, the container configured to maintain the interior volume in a sterile condition;
at least one electrical contact disposed within the interior volume of the base; and
an external charging portion, the external charging portion operably couplable to the container for charging a battery assembly positioned within the interior volume of the base via the at least one electrical contact.

2. The charging assembly according to claim 1, wherein the container further includes at least one charging bay disposed within the interior volume, the at least one charging bay configured to receive a battery assembly..

3. The charging assembly according to claim 1 or 2, wherein the base of the container is configured for wireless power transmission with the external charging portion and wherein the base of the container includes a support member incorporating an inductive coil and wherein the external charging portion includes an inductive charging mat, the support member and charging mat configured for wireless power transmission therebetween..

4. The charging assembly according to claim 1 or 2, wherein the container includes an exterior receptacle for coupling the container to the external charging portion and further comprising a connector cable, the connector cable configured to couple between the exterior receptacle of the container and the external charging portion for transmitting power therebetween.

5. The charging assembly according to any preceding claim, wherein the container is formed from a sterilizable material, and/or wherein the container is sterilizable via hydrogen peroxide sterilization.

6. A system, comprising:
a surgical instrument;
a battery assembly releasably engagable with the surgical instrument for powering the surgical instrument, the battery assembly being sterilizable; and
a charging assembly, including:
a container including a base configured to receive the battery assembly and a cover releasably engagable with the base for enclosing the battery assembly within the container, the container configured to maintain the battery assembly in a sterile condition; and
an external charging portion, the external charging portion operably couplable to the container for charging the battery assembly.

7. The system according to claim 6, wherein the battery assembly includes at least one electrical contact through which the battery assembly is charged and discharged, and preferably further comprising at least one electrical contact disposed within the container, the at least one electrical contact configured to couple to the at least one electrical contact of the battery assembly for charging the battery assembly.

8. The system according to claim 6 or 7, wherein the container includes a charging bay configured to receive the battery assembly.

9. The charging assembly according to claim 1 or 2 or the system according to claim 6, 7 or 8, wherein the base of the container is configured for wireless power transmission with the external charging portion.

10. The charging assembly according to claim 1 or 2 or the system according to claim 6, 7 or 8, wherein the container includes an exterior receptacle for coupling the container to the external charging portion.

11. The system according to any one of claims 6 to 10, wherein the container and battery assembly are sterilizable with the battery assembly disposed within the container, and/or wherein the container and battery assembly are sterilizable via hydrogen peroxide sterilization.

12. A method, comprising: disengaging a battery assembly from a battery-powered surgical device;
sterilizing the battery assembly;
sterilizing a container;
enclosing the battery assembly within the container;
electrically coupling an external charging portion to the container for charging the battery assembly; and
returning the sterile battery assembly to a sterile environment.

13. The method according to claim 12, wherein the step of enclosing the battery assembly within the container is performed before sterilizing the battery assembly and sterilizing the container such that the battery assembly and container are sterilized with the battery assembly enclosed within the container.

14. The method according to claim 12 or 13, wherein the step of coupling the external charging portion to the container for charging the battery assembly includes approximating wireless power transmission components of the external charging portion and container relative to one another.

15. The method according to claim 12 or 13, wherein the step of coupling the external charging portion to the container for charging the battery assembly includes coupling a connector cable between the container and the external charging portion.
